# EUROPEAN PATENT SPECIFICATION

(11) **EP 1 925 262 B1**
(45) Date of publication and mention of the grant of the patent: **18.03.2015**
(21) Application number: 07121648.5
(22) Date of filing: 27.11.2007
(51) Int. Cl.: A61B 17/34, A61B 10/02, A61B 19/00

(54) **System for navigating a surgical needle toward an organ of the body of a patient**
System zur Navigation einer chirurgischen Nadel zu einem Organ im Körper eines Patienten
Procédé pour guider une aiguille chirurgicale vers un organe du corps d'un patient

(30) Priority: 27.11.2006 US 861118 P
(43) Date of publication of application: 28.05.2008
(73) Proprietor: Mediguide Ltd., 31053 Haifa (IL)
(72) Inventor: Sobe, Lior, 43583, Ra'anana (IL)
(74) Representative: Kramer Barske Schmidtchen Patentanwälte PartG mbB

(56) References cited:
- EP-A- 1 184 684
- EP-A- 1 374 791
- WO-A-97/29682
- GB-A- 2 423 369
- US-A- 4 431 006
- US-A1- 2005 277 829

## Description

### FIELD OF THE DISCLOSED TECHNIQUE

The disclosed technique relates to medical devices in general, and to systems for withdrawal of a fluid sample from the body of a patient, in particular.

### BACKGROUND OF THE DISCLOSED TECHNIQUE

In order to diagnose a disease in a patient, a sample of an organic substance, such as a tissue or amniotic fluid is removed from the body of the patient. In case of a solid substance, such as a target tissue, the sample is removed from the body, by employing a biopsy needle. The biopsy needle includes a receptacle to remove the sample. In case of a fluid, such as amniotic fluid, a surgical needle is inserted into the uterus cavity of the uterus of the patient, and a sample of the amniotic fluid is pumped out through a lumen of the surgical needle. The mucous membrane of the vesical surface of the uterus cavity can block the opening of the surgical needle, while the surgical needle passes through the vesical surface to enter the uterus cavity.

A mandrel is inserted in the lumen, when the surgical needle is inserted into the body of the patient, in order to block the opening of the surgical needle, and to prevent entry of undesired tissues and fluids, into the lumen, and thereby prevent contamination of the sample. When the tip of the surgical needle reaches the desired location within the uterus cavity, the mandrel is pulled out of the lumen, and the sample of the amniotic fluid is pumped out. It is desirable for the physical staff to know the location and orientation of the tip of the surgical needle within the body of the patient, in order to minimize physical injury to the tissues surrounding the desired organ.

Methods for determining the location and orientation of the tip of a surgical device, such as a catheter, or a biopsy needle are known in the art. One such method utilizes a sensor wound around the tip of the biopsy needle. The sensor produces an electrical output in response to an electromagnetic filed, according to the location and orientation of the sensor in space. A display displays a representation of the location and orientation of the tip of the biopsy needle, superimposed on an image of the body of the patient, according to the output of the sensor.

Another method utilizes a sensor located within the tip of the catheter, and the sensor detects the location and orientation of the tip of the catheter in a similar manner. Yet another method, utilizes an electron spin resonance (ESR) sample placed within a probe which is inserted into the body of a patient, who is imaged by a magnetic resonance imaging (MRI) apparatus. The location and orientation of the ESR sample is determined according to the frequency of the ESR, in presence of a magnetic field of the MRI.

International Application Publication Number WO 97/29682 to Ben-Haim et al., and entitled "Locatable Biopsy Needle" is directed to a system for determining the trajectory of a biopsy needle while being advanced toward a target tissue within a body. The system includes an ultrasonic imager, a first position sensor, and a second position sensor. The biopsy needle includes an inner portion. The inner portion includes a tissue receptacle. The first position sensor is located distal to the tissue receptacle. The second position sensor is mounted on the ultrasonic imager.

The ultrasonic imager is placed on the body above the target tissue. An image plane of the ultrasonic imager bisects the target tissue. The position of the first position sensor relative to the image plane, can be dynamically determined. The actual trajectory over which biopsy needle advances can be determined by storing the positions of the needle during its movement. The preamble of appended claim 1 is based on this disclosure.

US Patent No. 6,073,043 issued to Schneider and entitled "Measuring Position and Orientation Using Magnetic Fields", is directed to a system for determining the position and orientation of a catheter. The system includes a plurality of field generation means, a sensor, an amplifier, an analog to digital converter (ADC), a processor, a digital to analog converter (DAC), a multiplexer, and a plurality of driving amplifiers.

Each field generating means includes a pair of B-field generator coils (i.e., magnetic field coils). The sensor is in the form of a coil. The signal processor is in the form of a low pass filter to reduce out of band signals to reach the processor. The sensor is connected to the amplifier. The signal processor is connected to the amplifier and to the ADC. The processor is connected to the ADC and to the DAC. The multiplexer is connected to the DAC and to the driving amplifiers. The driving amplifiers are connected to the field generating means.

The driving amplifiers supply power to each of the B-field generator coils. The sensor receives electromagnetic fields which the field generating means generates. The amplifier amplifies an output of the sensor. The signal processor processes the amplified output of the amplifier. The ADC converts the amplified output from analog to digital format. The processor determines the position and orientation of the sensor, by performing a signal withdrawal method.

US Patent No. 5,882,304 issued to Ehnholm et al., and entitled "Method and Apparatus for Determining Probe Location", is directed to a system for determining the position of a probe within an anatomy of a patient. The system includes a probe, the lock unit, a position acquisition controller, a gradient controller, and a display. The probe includes an active electron spin resonance (ESR) sample, which exhibits resonance when located in a magnetic field produced by a magnet of a magnetic resonance imaging (MRI) apparatus. The MRI apparatus includes the magnet and a main magnetic field and gradient coils. The lock unit is connected to the probe and to the position acquisition controller. The position acquisition controller is connected to the gradient controller. The display is connected to the position acquisition controller.

The patient is placed in an imaging region of the MRI apparatus. The probe is inserted into a biopsy needle and the biopsy needle is inserted into the anatomy of the patient. The lock unit measures the ESR frequency of the ESR sample, and the local field which acts on the probe. The position acquisition controller acts on the gradient coils through the gradient controller, according to the result of this measurement.

The MRI apparatus produces three gradient magnetic fields. The position of the ESR sample is determined according to the three gradient magnetic fields. In the presence of one of the gradient magnetic fields, the ESR frequency is a function of the position of the ESR sample along that gradient magnetic field. The system determines the coordinates of the probe, by measuring the ESR frequency in three directions. The display displays the position of the probe superimposed on an image of the patient. When the biopsy needle reaches the desired position in the anatomy of the patient, the probe is removed from the biopsy needle, and a biopsy mandrel is inserted in the biopsy needle, in order to withdraw a biopsy sample from the anatomy of the patient.

GB 2 423 369 A relates to a probe for generating position data for use in computer assisted surgery, which comprises a handle, a shaft extending from the handle, a tip attached to the shaft, the tip having a distal end for contacting an object to indicate the position of the object, and a sensor responsive to external electromagnetic fields for generating position data with multiple degrees of freedom, wherein the sensor is located within the shaft towards the distal end of the shaft.

EP 1 184 684 A2 discloses a method for calibrating a medical system capable of generating a magnetic field for tracking a position of a medical device.

US 4,431,006 A discloses a passive ultrasound needle probe locator.

EP 1 374 791 A1 relates to a position sensing system with integral location pad and position display.

### SUMMARY OF THE DISCLOSED TECHNIQUE

It is an object of the disclosed technique to provide a surgical needle device and a system for navigating a surgical needle toward a target organ of the body of a patient.

In accordance with the present invention, there are thus provided a surgical needle device according to claim 1 and a system for navigating a surgical needle toward a target organ of the body of a patient according to claim 15.

### BRIEF DESCRIPTION OF THE DRAWINGS

The disclosed technique will be understood and appreciated more fully from the following detailed description taken in conjunction with the drawings in which:
Figure 1 is a schematic illustration of a system for navigating a surgical needle toward a target organ of the body of a patient, constructed and operative according to an embodiment of the disclosed technique;
Figure 2 is a schematic illustration of a system for navigating a surgical needle toward a target organ of the body of a patient, constructed and operative according to another embodiment of the disclosed technique;
Figure 3 is a schematic illustration of a device for either withdrawing a sample of a bodily fluid from a target organ of the body of a patient, or injecting a therapeutic substance into the target organ, constructed and operative according to a further embodiment of the disclosed technique;
Figure 4 is a schematic illustration of a device, either for withdrawing a sample of a bodily fluid from a target organ of the body of a patient, or injecting a therapeutic substance into the target organ, constructed and operative according to another embodiment of the disclosed technique;
Figure 5 is a schematic illustration of a device either for withdrawing a sample of a bodily fluid from a target organ of the body of a patient, or injecting a therapeutic substance into the target organ, constructed and operative according to a further embodiment of the disclosed technique; and
Figure 6 is a schematic illustration of a method for operating the system of Figure 1.

### DETAILED DESCRIPTION OF THE EMBODIMENTS

The disclosed technique overcomes the disadvantages of the prior art by providing a medical positioning system (MPS) sensor located at the tip of a removable mandrel of a surgical needle, and an MPS coupled with the MPS sensor and with an electromagnetic field generator. The surgical needle is employed either to withdraw a sample of a bodily fluid of a target organ of the body of a patient, or to inject a therapeutic substance (e.g., anticarcinogen, anticoagulant) into the target organ. The removable mandrel can be moved in and out of the surgical needle, and it blocks the entrance of undesired bodily substances of the body of a patient, into the surgical needle, while the surgical needle is being advanced into the body of the patient, toward the target organ.

The MPS sensor produces an output according to the electromagnetic field which the electromagnetic field generator generates. The MPS determines the coordinates of the tip of the removable mandrel, in a coordinate system respective of the MPS, according to the output of the MPS sensor. The MPS superimposes a representation of the tip of the removable mandrel, on an image of the target organ, according to the coordinates of the tip of the removable mandrel, to enable withdrawal of the sample of the bodily fluid from the organ, after the removable mandrel is moved out of the surgical needle. The term "position" herein below, refers either to the location, to the orientation or both the location and the orientation, of an object in a three-dimensional coordinate system.

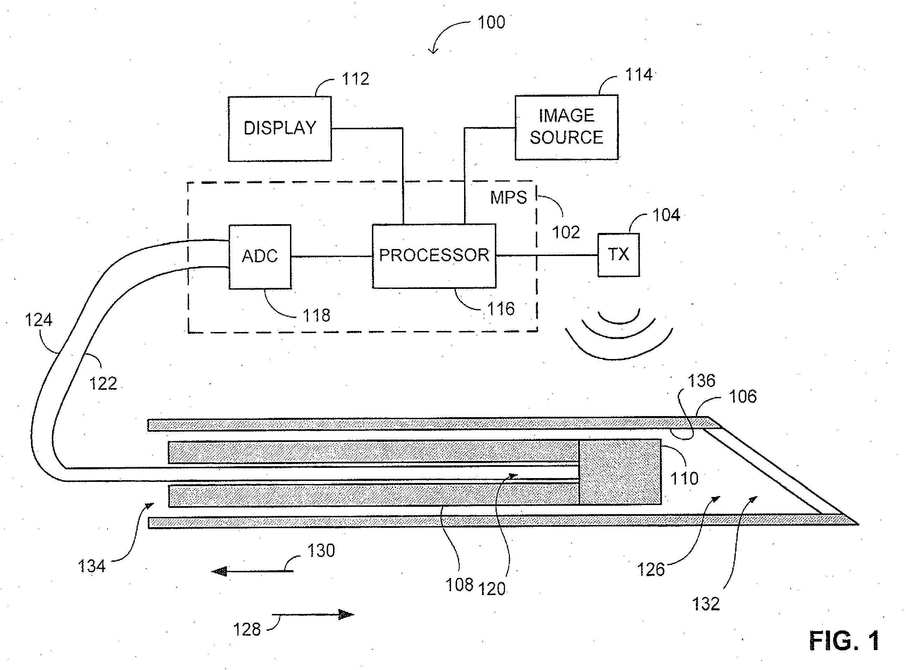

Reference is now made to Figure 1, which is a schematic illustration of a system, generally referenced 100, for navigating a surgical needle toward a target organ of the body of a patient, constructed and operative according to an embodiment of the disclosed technique. System 100 includes a medical positioning system (MPS) 102, a magnetic field generator 104, a surgical needle 106, a removable mandrel 108, an MPS sensor unit 110, a display 112, and an image source 114. MPS 102 includes a processor 116, and an analog to digital converter (ADC) 118. MPS sensor unit 110 includes an MPS housing (not shown) and an MPS sensor (not shown). The MPS housing can be in form of an adhesive applied over MPS sensor unit 110, plastic tube, elastomeric tube over MPS sensor unit 110 by applying heat, and the like. The MPS sensor is located within the MPS housing. The MPS housing is in the form of a cylinder. Processor 116 is coupled with electromagnetic field generator 104, display 112, image source 114, and with ADC 118.

The MPS sensor is in the form of an electromagnetic coil (i.e., a wound wire), which produces an electrical output in response to an electromagnetic field. The MPS housing is made of a metal, such as stainless steel, and the like. Removable mandrel 108 is made of a metal, such as stainless steel, plastic, ceramic, and the like. Removable mandrel 108 is in the form of a tube having a bore 120. MPS sensor unit 110 is firmly coupled with the tip of removable mandrel 108 by methods known in the art, such as welding, brazing, employing an adhesive, pressure fit (e.g., MPS sensor unit 110 having a conical shape), and the like. The MPS sensor is coupled with ADC 118 by wires 122 and 124, which pass through bore 120. Image source 114 is in the form of an imager such as computer tomography (CT), magnetic resonance imager (MRI), positron emission tomography (PET), single photon emission computer tomography (SPECT), ultrasound image detector, infrared image detector, X-ray imager (e.g., C-arm), optical coherence tomography (OCT), and the like. Image source 114 provides a real-time video image (not shown) of an organ (not shown) of the body (not shown) of a patient (not shown), which is acquired during medical operation on the patient. Alternatively, image source 114 is in the form of a database, which includes an image of the organ, which is acquired prior to the medical operation on the patient. Further alternatively, image source 114 includes a still image of the organ.

Image source 114 can produce a two-dimensional image of the target organ. Alternatively, image source 114 can produce a three-dimensional image of the target organ. Further alternatively, image source 114 can produce a right view and a left view of the target organ, thereby enabling a user to perceive a stereoscopic sensation of the image, by viewing the image on display 112 (e.g., by employing a stereoscopic pair of glasses).

An outer diameter of removable mandrel 108 is less than an inner diameter of a lumen 126 of surgical needle 106, to enable movement of removable mandrel 108 within lumen 126, in directions designated by arrows 128 and 130. The outer diameter of removable mandrel 108 and of MPS sensor unit 110 is of such value that MPS sensor unit 110 and removable mandrel 108 can be moved in unison, in directions 128 and 130, while MPS sensor unit 110 physically separates a distal portion 132 of lumen 126 from a proximal portion 134 of lumen 126. In this manner, MPS sensor unit 110 seals against an inner wall 136 of lumen 126, and thereby, fluids and solid materials which are located at distal portion 132, can not reach proximal portion 134.

The user can employ surgical needle 106 to withdraw a sample of a bodily fluid (e.g., amniotic fluid) from a target organ of the patient (e.g., the uterus cavity of the patient). Alternatively, the user can employ surgical needle 106 to inject a therapeutic substance (e.g., anticarcinogen, anticoagulant), into the target organ. In case surgical needle 106 is employed for collecting a fluid sample, removable mandrel 108 is employed for preventing contamination of the fluid sample. In case surgical needle 106 is employed for injecting a therapeutic substance into the target organ, removable mandrel 108 is employed for flushing out chemical compounds from surgical needle 106.

When the user pierces the skin of the patient with surgical needle 106, in order to reach a selected region of the target organ, which includes the desired bodily fluid, surgical needle 106 passes through various tissues and fluids, which are located in the vicinity of the target organ. In order to perform a reliable assay of the bodily fluid of the target organ, the sample of the bodily fluid should be substantially pure and substantially free of undesired bodily substances (e.g., tissues and fluids) which are located in the vicinity of the target organ.

Before piercing the skin of the patient, the user moves removable mandrel 108 in direction 128 into lumen 126 of surgical needle 106, such that MPS sensor unit 110 is located at distal portion 132. In this manner, MPS sensor unit 110 prevents the undesired substances to reach proximal portion 134 from distal portion 132, and blocks entrance of the undesired substances to proximal portion 134.

The MPS sensor produces an analog electrical output in response to the electromagnetic filed which electromagnetic filed generator 104 generates. ADC 118 converts the analog electrical output to a digital format, and provides this digital output to processor 116. Processor 116 determines the position of the MPS sensor, and thus the tip of removable mandrel 108 in a three-dimensional coordinate system, according to this digital output. Processor 116 produces an indication of the position of the tip of surgical needle 106 according to the position of the MPS sensor, for the user to navigate surgical needle 106 toward the target organ. This indication can be for example, visual, aural, tactile, and the like.

In case of a visual indication, display 112 displays the visual indication. In case the indication is aural, or tactile, the system includes a user interface (not shown), coupled with the processor, to present this indication to the user.

Processor 116 can superimpose a representation (not shown) of the position of the tip of removable mandrel 108, on an image of the target organ which image source 114 provides. Processor 116, then directs display 112 to display a superposition of the representation of the position of the tip of removable mandrel 108 on the image of the target organ. In this manner, the user can view a trajectory of the tip of removable mandrel 108, and distal portion 132, as the user advances surgical needle 106 in the body of the patient, toward the target organ. With the aid of this view, the user can maneuver surgical needle 106 within the body of the patient, in such a manner that the surrounding tissue is minimally severed, and furthermore, the distal portion 132 reaches directly the selected region of the target organ.

The user can employ surgical needle 106 to withdraw a sample of a bodily fluid from the target organ. In this case, when system 100 informs the user that distal portion 132 is located at the selected region of the target organ, the user can pull out removable mandrel 108 from lumen 126 of surgical needle 106, and collect the sample of the bodily fluid in a container (e.g., a vial), by employing a sucking mechanism (e.g., a mechanical pump, an electric pump). It is noted that MPS sensor unit 110 which is located at the tip of removable mandrel 108, blocks entrance of undesired bodily substances to proximal portion 134, thereby preventing contamination of the sample of the bodily fluid of the target organ.

Alternatively, the user can employ the surgical needle to inject a therapeutic substance into the target organ. In this case, when the system informs the user that distal portion of the surgical needle is located at the selected region of the target organ, the user can pull out a removable mandrel which is made of a solid rod, from the lumen of the surgical needle, and then inject the therapeutic substance into the target organ.

It is further noted that surgical needle 106 can be a disposable surgical needle in order to prevent transfer of contagious diseases among different patients. However, removable mandrel 108 together with sensor unit 110 can be used for performing medical operations on different patients. In this case, the probability of transfer of a virus or a bacterium among patients is reduced, for example, by placing a disposable barrier over the removable mandrel (e.g., a polymer sheet such as Latex), by sterilizing the removable mandrel prior to the medical operation, and the like.

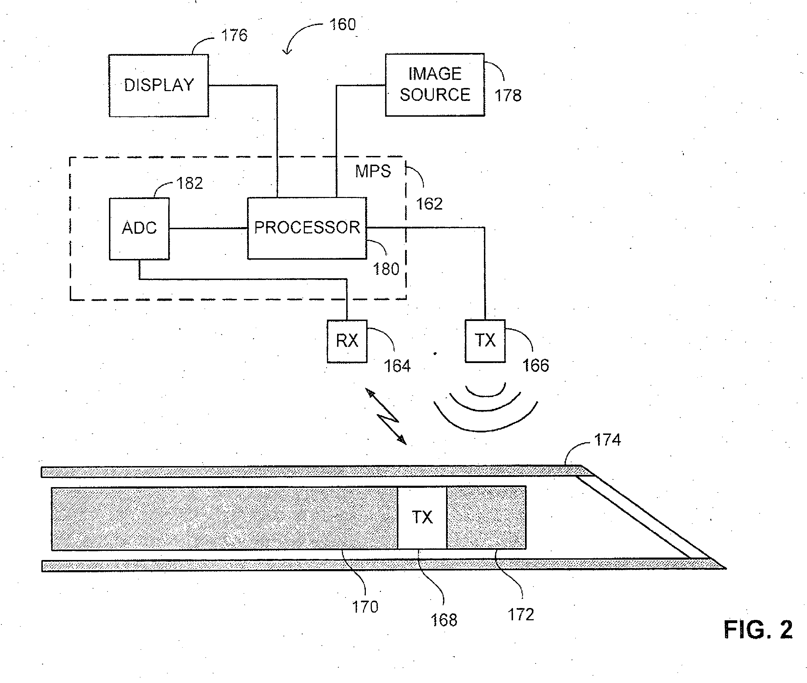

Reference is now made to Figure 2, which is a schematic illustration of a system, generally referenced 160, for navigating a surgical needle toward a target organ of the body of a patient, constructed and operative according to another embodiment of the disclosed technique. System 160 includes an MPS 162, a receiver 164, an electromagnetic field generator 166, a transmitter 168, a removable mandrel 170, an MPS sensor unit 172, a surgical needle 174, a display 176 and an image source 178. MPS 162 includes a processor 180 and an ADC 182. MPS 162, electromagnetic field generator 166, surgical needle 174, display 176 and image source 178, are similar to MPS 102, electromagnetic field generator 104, surgical needle 106, display 112, and image source 114, respectively, as described herein above in connection with Figure 1.

MPS sensor unit 172 includes an MPS sensor (not shown) and an MPS housing (not shown), similar to the MPS sensor and the MPS housing of sensor unit 110, as described herein above in connection with Figure 1. Removable mandrel 170 is in the form of a solid rod. Alternatively, the removable mandrel is in the form of a tube, similar to removable mandrel 108, as described herein above in connection with Figure 1.

Processor 180 is coupled with electromagnetic field generator 166, display 176, image source 178, and with ADC 182. Receiver 164 is coupled with ADC 182. MPS sensor unit 172 is coupled with the tip of removable mandrel 170 in a similar manner of coupling of MPS sensor unit 110 with removable mandrel 108, as described herein above in connection with Figure 1. Transmitter 168 is coupled with receiver 164 by a wireless link, such as Bluetooth, WiFi, Zigbee, IEEE 802 series connections, and the like.

Transmitter 168 is physically coupled with removable mandrel 170 and with MPS sensor unit 172, and electrically coupled with the MPS sensor. Transmitter 168 is located at the tip of removable mandrel 170. In this case, removable mandrel 170 is in the form of a solid rod. Alternatively, transmitter 168 is located at a proximal end of the removable mandrel, in which case the transmitter is coupled with the MPS sensor by a pair of wires which pass through a bore of the removable mandrel. Further alternatively, transmitter 168 can be integrated with MPS sensor unit 172. System 160 operates similar to system 100, except that the MPS sensor is coupled with MPS 162 by a wireless link.

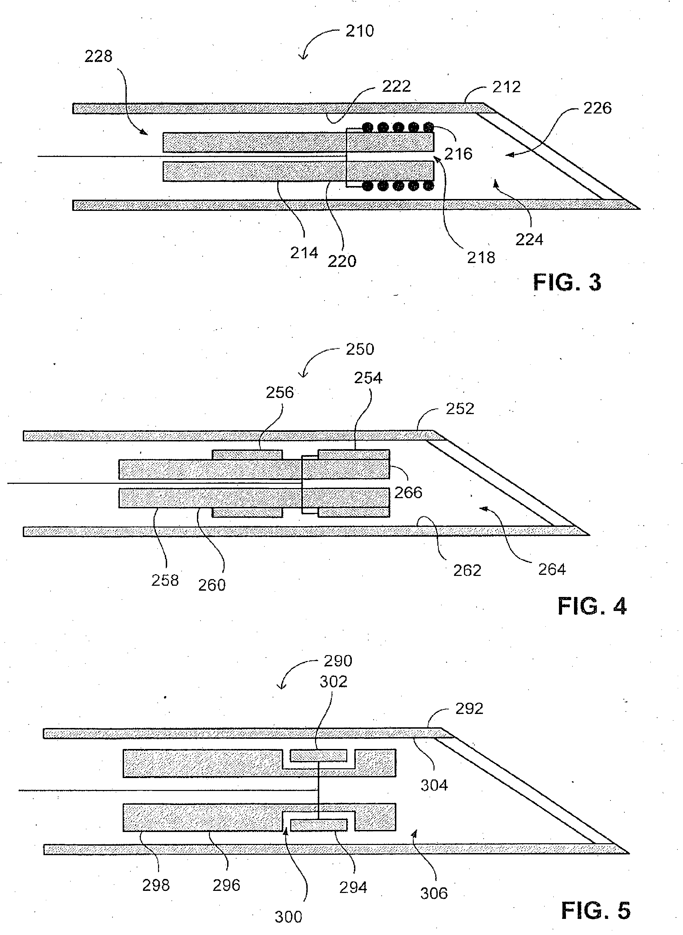

Reference is now made to Figure 3, which is a schematic illustration of a device, generally referenced 210, for either withdrawing a sample of a bodily fluid from a target organ of the body of a patient, or injecting a therapeutic substance into the target organ, constructed and operative according to a further embodiment of the disclosed technique. Device 210 includes a surgical needle 212, a removable mandrel 214 and an MPS sensor 216. Removable mandrel 214 is in the form of a tubing, having a bore 218. MPS sensor 216 is in the form of a wire, which is wound around an outer surface 220 of removable mandrel 214, at the tip of removable mandrel 214. MPS sensor 216 is coupled with an MPS (not shown) similar to MPS 102 (Figure 1), with a pair of wires (not shown) passing through bore 218, as described herein above.

Alternatively, device 210 can include a transmitter (not shown), similar to transmitter 168 (Figure 2) as described herein above. This transmitter is coupled with the MPS sensor, with the removable mandrel, and with a receiver (not shown), similar to the coupling as described herein above in connection with Figure 2.

Each of an outer diameter of removable mandrel 214, and a wire diameter of MPS sensor 216 is of such value that MPS sensor 216 and outer surface 220 of removable mandrel 214 seal against an inside wall 222 of a lumen 224 of surgical needle 212. In this manner, the tip of removable mandrel 214 blocks entrance of undesired bodily substances from a distal portion 226 of lumen 224 to a proximal portion 228 of lumen 224. Hence, device 210 enables withdrawal of a substantially uncontaminated sample of a bodily fluid from a target organ (not shown) of the body (not shown) of a patient (not shown).

In the example set forth in Figure 3, removable mandrel 214 is in the form of a tubing. Applicant has found out that if the diameter of bore 218 is small enough, then removable mandrel 214 can block the entrance of undesired bodily substances from distal portion 226 of lumen 224 to proximal portion 228 of lumen 224. It is noted that this blocking action depends on the relation between the diameter of bore 218 and the viscosity of the undesired bodily substances (i.e., if the diameter of bore 218 is sufficiently small, or the viscosity of the undesired bodily substance is sufficiently large, then the undesired bodily substance can not flow within bore 218). However, the removable mandrel can be made of a solid rod, in which case MPS sensor 216 sends an output thereof to the MPS, via the transmitter.

Reference is now made to Figure 4, which is a schematic illustration of a device generally referenced 250, either for withdrawing a sample of a bodily fluid from a target organ of the body of a patient, or injecting a therapeutic substance into the target organ, constructed and operative according to another embodiment of the disclosed technique. Device 250 includes a surgical needle 252, an MPS sensor unit 254, a radiopaque marker 256 and a removable mandrel 258. MPS sensor unit 254 includes an MPS sensor (not shown) and an MPS housing (not shown). The MPS sensor unit is located within the MPS housing. MPS sensor unit 254 and radiopaque marker 256 are located at a distal portion 266 of removable mandrel 258.

The MPS housing includes a housing bore there within (i.e., the MPS housing is in the form of a tube). An inner diameter of the housing bore is substantially equal to an outer diameter of removable mandrel 258. An inside wall (not shown) of the housing bore is coupled with an outer surface 260 of removable mandrel 258, by fastening methods known in the art, such as welding, brazing, by employing an adhesive, and the like. Radiopaque marker 256 is in the form of a metallic foil, which is visible in an X-ray image thereof (i.e., radiopaque marker 256 fluoresces under X-ray). Each of a housing outside diameter of the MPS housing, an inside wall diameter of an inside wall 262 of a lumen 264 of surgical needle 252, and a marker outer diameter of radiopaque marker 256 is of such value that MPS sensor unit 254 seals against inside wall 262, while an assembly of removable mandrel 258, MPS sensor unit 254 and radiopaque marker 256 move within lumen 264.

The MPS sensor is coupled with an MPS (not shown). A processor (not shown) superimposes a representation of a position of the tip of removable mandrel 258 on a real-time image (e.g., an X-ray image - not shown) of a target organ (not shown) of the body (not shown) of a patient (not shown). The processor directs a display (not shown) to display this X-ray image, along with a real-time image of radiopaque marker 256.

In the example set forth in Figure 4, removable mandrel 258 is in the form of a tubing. Applicant has found out that if the diameter of a mandrel bore of removable mandrel 258 is small enough, then removable mandrel 258 can block the entrance of undesired bodily substances from a distal portion of lumen 264 to a proximal portion of lumen 264. However, the removable mandrel can be made of a solid rod, in which case the MPS sensor sends an output thereof to the MPS, via a transmitter (not shown), similar to transmitter 168 (Figure 2) as described herein above.

Reference is now made to Figure 5, which is a schematic illustration of a device generally referenced 290, either for withdrawing a sample of a bodily fluid from a target organ of the body of a patient, or injecting a therapeutic substance into the target organ, constructed and operative according to a further embodiment of the disclosed technique. Device 290 includes a surgical needle 292, an MPS sensor unit 294 and a removable mandrel 296. MPS sensor unit 294 includes an MPS sensor (not shown) and an MPS housing (not shown). The MPS sensor is located within the MPS housing.

A mandrel outer surface 298 of removable mandrel 296 includes an undercut 300. MPS sensor unit 294 is similar to MPS sensor unit 254 (Figure 4) as described herein above. MPS sensor unit 294 fits inside undercut 300. Each of a housing outer diameter of the MPS housing, and a mandrel outer diameter of removable mandrel 296 is of such value that mandrel outer surface 298 and a housing outer surface 302 of the MPS housing seal against an inside wall 304 of a lumen 306 of surgical needle 292, while removable mandrel 296 moves within lumen 306.

In the example set forth in Figure 5, removable mandrel 296 is in the form of a tubing. Applicant has found out that if the diameter of a mandrel bore of removable mandrel 296 is small enough, then removable mandrel 296 can block the entrance of undesired bodily substances from a distal portion of lumen 306 to a proximal portion of lumen 306. However, the removable mandrel can be made of a solid rod, in which case the MPS sensor sends an output thereof to the MPS, via a transmitter (not shown), similar to transmitter 168 (Figure 2) as described herein above.

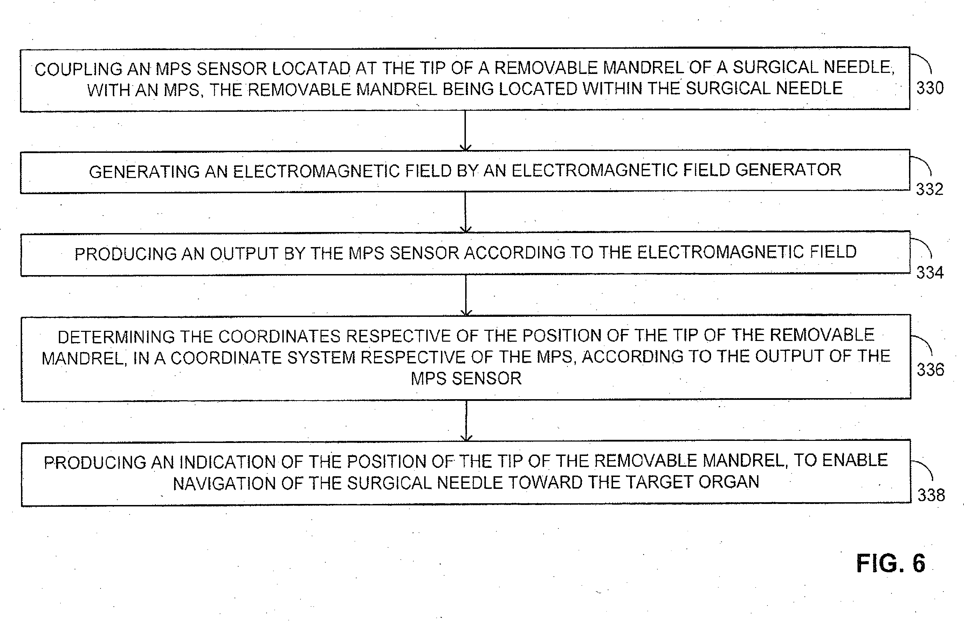

Reference is now made to Figure 6, which is a schematic illustration of a method for operating the system of Figure 1. In procedure 330, an MPS sensor located at the tip of a removable mandrel of a surgical needle, is coupled with an MPS, the removable mandrel being located within the surgical needle. With reference to Figure 1, the MPS sensor of MPS sensor unit 110 is coupled with MPS 102, by wires 122 and 124. MPS sensor unit 110 is located at the tip of removable mandrel 108.

In procedure 332, an electromagnetic field is generated by an electromagnetic field generator. With reference to Figure 1, electromagnetic field generator 104 generates an electromagnetic field.

In procedure 334, an output is produced by the MPS sensor according to the electromagnetic field. With reference to Figure 1, the MPS sensor of MPS sensor unit 110 produces an analog electrical output, in response to the electromagnetic field generated by electromagnetic field generator 104.

In procedure 336, the coordinates respective of the position of the tip of the removable mandrel is determined, in a coordinate system respective of the MPS, according to the output of the MPS sensor. With reference to Figure 1, ADC 118 converts the analog electrical output produced by the MPS sensor in procedure 334, to digital format. Processor 116 determines the position of the MPS sensor, and thus the position of the tip of removable mandrel 108, in an MPS coordinate system respective of MPS 102, according to the electrical output of the MPS sensor, in digital format.

In procedure 338, an indication of the position of the tip of the removable mandrel is produced, to enable navigation of the surgical needle toward the target organ. With reference to Figure 1, processor 116 superimposes a representation of the position of the tip of removable mandrel 108, in an MPS coordinate system of MPS 102, on an image of the target organ, and directs display 112 to display this superimposed image. By viewing the superimposed image on display 112, the physical staff can verify the position of the tip of removable mandrel 108, and thus the tip of surgical needle 106 relative to the selected region within the target organ. Once the physical staff ensures that the tip of surgical needle 106 is located at the desired position within the target organ, she can withdraw a sample of the bodily fluid from the target organ, after removing removable mandrel 108 from surgical needle 106.

It will be appreciated by persons skilled in the art that the disclosed technique is not limited to what has been particularly shown and described hereinabove. Rather the scope of the disclosed technique is defined only by the claims, which follow.

## Claims

1. Surgical needle device comprising:
a surgical needle (106, 174, 212, 252, 292) having a lumen (126, 224, 264, 306), wherein the lumen (126, 224, 264, 306) has an inner wall (136, 222, 262, 304),
a removable mandrel (108, 170, 214, 258, 296) suitable for being located within said surgical needle (110, 172, 216, 254, 294), said removable mandrel (108, 170, 214, 258, 296) to be moved in and moved out of said surgical needle (106, 174, 212, 252, 292); and
a medical positioning system (MPS) sensor (110, 172, 216, 254, 294) located at the tip of said removable mandrel (108, 170, 214, 258, 296),
said MPS sensor (110, 172, 216, 254, 294) to be coupled with an MPS (102, 162), said MPS (102, 162) to be coupled with an electromagnetic field generator (104, 166), said electromagnetic field generator (104, 166) generating an electromagnetic field, said MPS sensor (110, 172, 216, 254, 294) producing an output according to said electromagnetic field, said MPS (102, 162) determining the position of said tip of said removable mandrel (108, 170, 214, 258, 296) in a coordinate system respective of said MPS (102, 162), according to said output of said MPS sensor (110, 172, 216, 254, 294), said MPS (102, 162) producing an indication respective of said position, to enable navigation of said surgical needle (106, 174, 212, 252, 292) toward a target organ,
**characterized in that**
said MPS sensor (110, 172, 216, 254, 294) is firmly coupled with the tip of said removable mandrel (108, 170, 214, 258, 296) such that said MPS sensor (110, 172, 216, 254, 294) seals against said inner wall (136, 222, 262, 304) of said lumen (126, 224, 264, 306), whereby the entrance of undesired bodily substances into the surgical needle (106, 174, 212, 252, 292) is blocked, while said surgical needle (106, 174, 212, 252, 292) is being advanced toward said target organ.

2. The device according to claim 1, wherein said surgical needle (106, 174, 212, 252, 292) is suitable for withdrawing a sample of a bodily fluid from said target organ.

3. The device according to claim 1 or 2, wherein said surgical needle (106, 174, 212, 252, 292) is suitable for injecting a therapeutic substance into said target organ.

4. The device according to any one of the preceding claims, wherein said surgical needle is disposable.

5. The device according to any one of the preceding claims, wherein said MPS sensor is located within an MPS housing, said MPS housing being in the form of a cylinder,
wherein said MPS housing is firmly coupled with said tip of said removable mandrel (108, 170, 214, 258, 296).

6. The device according to any one of claims 1 to 4, wherein said MPS sensor is located within an MPS housing, said MPS housing being in the form of a tube, and
wherein said MPS housing is firmly coupled with an outer periphery of said removable mandrel (108, 170, 214, 258, 296), at a distal end of said removable mandrel (108, 170, 214, 258, 296).

7. The device according to claim 6, wherein an inner diameter of said MPS housing is substantially equal to an outer diameter of said removable mandrel (108, 170, 214, 258, 296).

8. The device according to claim 6, wherein a housing outer diameter (302) of said MPS housing is substantially equal to a mandrel outer diameter of said removable mandrel (108, 170, 214, 258, 296),
wherein said removable mandrel (108, 170, 214, 258, 296) includes an undercut (300) at a distal portion thereof, and
wherein said MPS housing is located within said undercut (300).

9. The device according to any one of claims 1 to 4, wherein said MPS sensor (110, 172, 216, 254, 294) is located within an MPS housing, said MPS housing being in the form of an adhesive, covering said MPS sensor (110, 172, 216, 254, 294).

10. The device according to any one of the preceding claims, wherein said removable mandrel (108, 170, 214, 258, 296) is in the form of a solid rod.

11. The device according to any one of claims 1 to 9, wherein said removable mandrel (108, 170, 214, 258, 296) is in the form of a tube.

12. The device according to any one of claims 1 to 4, wherein said MPS sensor is in the form of an electrically conductive coil wound around an outer surface of said removable mandrel (108, 170, 214, 258, 296).

13. The device according to any one of the preceding claims, further comprising a disposable barrier placed over said removable mandrel (108, 170, 214, 258, 296), said disposable barrier reducing the probability of transfer of a virus or a bacterium from said patient to another patient.

14. The device according to any one of the preceding claims, wherein said removable mandrel (108, 170, 214, 258, 296) blocks entrance of undesired bodily substances of said body of said patient, into said surgical needle (106, 174, 212, 252, 292), while said surgical needle (106, 174, 212, 252, 292) is being advanced toward said target organ.

15. System (100, 160) for navigating a surgical needle (106, 174, 212, 252, 292) toward a target organ of the body of a patient, the system (100, 160) comprising:
a surgical needle device according to any one of the preceding claims;
an electromagnetic field generator (104, 166) for generating an electromagnetic field; and
an MPS (102, 162) coupled with said MPS sensor (110, 172, 216, 254, 294) and with said electromagnetic field generator (104, 166), said MPS sensor (110, 172, 216, 254, 294) producing an output according to said electromagnetic field, said MPS (102, 162) determining the position of said tip of said removable mandrel (108, 170, 214, 258, 296) in a coordinate system respective of said MPS (102, 162), according to said output of said MPS sensor (110, 172, 216, 254, 294), said MPS (102, 162) producing an indication respective of said position, to enable navigation of said surgical needle (106, 174, 212, 252, 292) toward said target organ.

16. The system (100, 160) according to claim 15, further comprising:
a processor (116, 180) coupled with said MPS sensor (110, 172, 216, 254, 294);
an image source (114) coupled with said processor (116, 180), said image source (114) including an organ image of said target organ; and
a display (112) coupled with said processor (116, 180),
wherein said processor (116, 180) determines said position of said MPS sensor (110, 172, 216, 254, 296), according to said output,
wherein said processor (116, 180) produces a superposition of a representation of said position on said organ image, and
wherein said display (112) displays said superposition.

17. The system (100, 160) according to claim 16, further comprising a radiopaque marker (256) located at said tip of said removable mandrel (108, 170, 214, 258, 296), wherein said display (112) displays a marker image of said radiopaque marker (256) against an organ image of said target organ.

18. The system (100, 160) according to any one of claims 15 to 17, wherein said MPS sensor (110, 172, 216, 254, 294) is coupled with said MPS (102, 162), via an electric conductor.

19. The system (100, 160) according to any one of claims 15 to 17, wherein said indication is selected from the list consisting of:
visual, aural and tactile.

## Patentansprüche

1. Chirurgische Nadelvorrichtung, umfassend:
eine chirurgische Nadel (106, 174, 212, 252, 292) mit einem Lumen (126, 224, 264, 306), wobei das Lumen (126, 224, 264, 306) eine Innenwand (136, 222, 262, 304) aufweist,
einen entfernbaren Dorn (108, 170, 214, 258, 296), der zum Anordnen innerhalb der chirurgischen Nadel (110, 172, 216, 254, 294) geeignet ist, wobei der entfernbare Dorn (108, 170, 214, 258, 296) in die chirurgische Nadel hineinbewegt und aus der chirurgischen Nadel (106, 174, 212, 252, 292) herausbewegt werden soll, und
einen medizinisches Positionierungssystem (MPS)-Sensor (110, 172, 216, 254, 294), der an der Spitze des entfernbaren Dorns (108, 170, 214, 258, 296) angeordnet ist,
wobei der MPS-Sensor (110, 172, 216, 254, 294) mit einem MPS (102, 162) gekoppelt werden soll, wobei das MPS (102, 162) mit einer Einrichtung zur Erzeugung eines elektromagnetischen Felds (104, 166) gekoppelt werden soll, wobei die Einrichtung zur Erzeugung eines elektromagnetischen Felds (104, 166) ein elektromagnetisches Feld erzeugt, wobei der MPS-Sensor (110, 172, 216, 254, 294) ein Ausgangssignal gemäß dem elektromagnetischen Feld erzeugt, wobei das MPS (102, 162) die Position der Spitze des entfernbaren Dorns (108, 170, 214, 258, 296) in einem Koordinatensystem in Bezug auf das MPS (102, 162) gemäß dem Ausgangssignal des MPS-Sensors (110, 172, 216, 254, 294) bestimmt, wobei das MPS (102, 162) eine Angabe bezüglich der Position erzeugt, so dass eine Navigation der chirurgischen Nadel (106, 174, 212, 252, 292) in die Richtung eines Zielorgans ermöglicht wird,
**dadurch gekennzeichnet, dass**
der MPS-Sensor (110, 172, 216, 254, 294) fest mit der Spitze des entfernbaren Dorns (108, 170, 214, 258, 296) gekoppelt ist, so dass der MPS-Sensor (110, 172, 216, 254, 294) gegen die Innenwand (136, 222, 262, 304) des Lumens (126, 224, 264, 306) abdichtet, wodurch das Eintreten von unerwünschten Körpersubstanzen in die chirurgische Nadel (106, 174, 212, 252, 292) blockiert wird, während die chirurgische Nadel (106, 174, 212, 252, 292) in die Richtung des Zielorgans vorgeschoben wird.

2. Vorrichtung nach Anspruch 1, bei der die chirurgische Nadel (106, 174, 212, 252, 292) zum Entnehmen einer Probe eines Körperfluids von dem Zielorgan geeignet ist.

3. Vorrichtung nach Anspruch 1 oder 2, bei der die chirurgische Nadel (106, 174, 212, 252, 292) zum Injizieren einer therapeutischen Substanz in das Zielorgan geeignet ist.

4. Vorrichtung nach einem der vorhergehenden Ansprüche, bei der die chirurgische Nadel ein Wegwerfartikel ist.

5. Vorrichtung nach einem der vorhergehenden Ansprüche, bei der sich der MPS-Sensor innerhalb eines MPS-Gehäuses befindet, wobei das MPS-Gehäuse in der Form eines Zylinders vorliegt,
wobei das MPS-Gehäuse fest mit der Spitze des entfernbaren Dorns (108, 170, 214, 258, 296) gekoppelt ist.

6. Vorrichtung nach einem der Ansprüche 1 bis 4, bei der sich der MPS-Sensor innerhalb eines MPS-Gehäuses befindet, wobei das MPS-Gehäuse in der Form einer Röhre vorliegt, und
wobei das MPS-Gehäuse an einem distalen Ende des entfernbaren Dorns (108, 170, 214, 258, 296) fest mit einem Außenumfang des entfernbaren Dorns (108, 170, 214, 258, 296) gekoppelt ist.

7. Vorrichtung nach Anspruch 6, bei der ein Innendurchmesser des MPS-Gehäuses im Wesentlichen gleich einem Außendurchmesser des entfernbaren Dorns (108, 170, 214, 258, 296) ist.

8. Vorrichtung nach Anspruch 6, bei der ein Gehäuseaußendurchmesser (302) des MPS-Gehäuses im Wesentlichen gleich einem Dornaußendurchmesser des entfernbaren Dorns (108, 170, 214, 258, 296) ist,
wobei der entfernbare Dorn (108, 170, 214, 258, 296) eine Ausnehmung (300) an einem distalen Abschnitt davon umfasst, und
wobei sich das MPS-Gehäuse innerhalb der Ausnehmung (300) befindet.

9. Vorrichtung nach einem der Ansprüche 1 bis 4, bei der sich der MPS-Sensor (110, 172, 216, 254, 294) innerhalb eines MPS-Gehäuses befindet, wobei das MPS-Gehäuse in der Form eines Haftmittels vorliegt, das den MPS-Sensor (110, 172, 216, 254, 294) bedeckt.

10. Vorrichtung nach einem der vorhergehenden Ansprüche, bei welcher der entfernbare Dorn (108, 170, 214, 258, 296) in der Form eines massiven Stabs vorliegt.

11. Vorrichtung nach einem der Ansprüche 1 bis 9, bei welcher der entfernbare Dorn (108, 170, 214, 258, 296) in der Form einer Röhre vorliegt.

12. Vorrichtung nach einem der Ansprüche 1 bis 4, bei welcher der MPS-Sensor in der Form einer elektrisch leitenden Spule vorliegt, die um eine Außenfläche des entfernbaren Dorns (108, 170, 214, 258, 296) gewunden ist.

13. Vorrichtung nach einem der vorhergehenden Ansprüche, die ferner eine Einwegbarriere umfasst, die über dem entfernbaren Dorn (108, 170, 214, 258, 296) angeordnet ist, wobei die Einwegbarriere die Wahrscheinlichkeit einer Übertragung eines Virus oder eines Bakteriums von einem Patienten zu einem anderen Patienten vermindert.

14. Vorrichtung nach einem der vorhergehenden Ansprüche, bei welcher der entfernbare Dorn (108, 170, 214, 258, 296) das Eintreten von unerwünschten Körpersubstanzen des Körpers des Patienten in die chirurgische Nadel (106, 174, 212, 252, 292) blockiert, während die chirurgische Nadel (106, 174, 212, 252, 292) in die Richtung des Zielorgans vorgeschoben wird.

15. System (100, 160) zum Navigieren einer chirurgischen Nadel (106, 174, 212, 252, 292) in die Richtung eines Zielorgans des Körpers eines Patienten, wobei das System (100, 160) umfasst:
eine chirurgische Nadelvorrichtung gemäß einem der vorhergehenden Ansprüche,
eine Einrichtung zur Erzeugung eines elektromagnetischen Felds (104, 166) zum Erzeugen eines elektrischen Felds, und
ein MPS (102, 162), das mit dem MPS-Sensor (110, 172, 216, 254, 294) und mit der Einrichtung zur Erzeugung eines elektromagnetischen Felds (104, 166) gekoppelt ist, wobei der MPS-Sensor (110, 172, 216, 254, 294) ein Ausgangssignal gemäß dem elektromagnetischen Feld erzeugt, wobei das MPS (102, 162) die Position der Spitze des entfernbaren Dorns (108, 170, 214, 258, 296) in einem Koordinatensystem in Bezug auf das MPS (102, 162) gemäß dem Ausgangssignal des MPS-Sensors (110, 172, 216, 254, 294) bestimmt, wobei das MPS (102, 162) eine Angabe bezüglich der Position erzeugt, so dass die chirurgische Nadel (106, 174, 212, 252, 292) in die Richtung des Zielorgans navigiert werden kann.

16. System (100, 160) nach Anspruch 15, ferner umfassend:
einen Prozessor (116, 180), der mit dem MPS-Sensor (110, 172, 216, 254, 294) gekoppelt ist,
eine Bildquelle (114), die mit dem Prozessor (116, 180) gekoppelt ist, wobei die Bildquelle (114) ein Organbild des Zielorgans umfasst, und
eine Anzeige (112), die mit dem Prozessor (116, 180) gekoppelt ist,
wobei der Prozessor (116, 180) die Position des MPS-Sensors (110, 172, 216, 254, 296) gemäß dem Ausgangssignal bestimmt,
wobei der Prozessor (116, 180) eine Überlagerung einer Darstellung der Position auf dem Organbild erzeugt und
wobei die Anzeige (112) die Überlagerung anzeigt.

17. System (100, 160) nach Anspruch 16, das ferner einen strahlenundurchlässigen Marker (256) umfasst, der sich an der Spitze des entfernbaren Dorns (108, 170, 214, 258, 296) befindet, wobei die Anzeige (112) ein Markerbild des strahlenundurchlässigen Markers (256) gegen ein Organbild des Zielorgans anzeigt.

18. System (100, 160) nach einem der Ansprüche 15 bis 17, bei dem der MPS-Sensor (110, 172, 216, 254, 294) mit dem MPS (102, 162) mittels eines elektrischen Leiters gekoppelt ist.

19. System (100, 160) nach einem der Ansprüche 15 bis 17, bei dem die Angabe aus der Gruppe, bestehend aus sichtbar, hörbar und fühlbar, ausgewählt ist.

## Revendications

1. Dispositif à aiguille chirurgicale comprenant :
une aiguille chirurgicale (106, 174, 212, 252, 292) présentant un lumen (126, 224, 264, 306), dans lequel le lumen (126, 224, 264, 306) présente une paroi intérieure (136, 222, 262, 304),
un mandrin démontable (108, 170, 214, 258, 296) adapté pour être situé dans ladite aiguille chirurgicale (110, 172, 216, 254, 294), ledit mandrin démontable (108, 170, 214, 258, 296) étant destiné à être déplacé dans et déplacé en dehors de ladite aiguille chirurgicale (106, 174, 212, 252, 292) ; et
une sonde (110, 172, 216, 254, 294) de système de positionnement médical (MPS) située au sommet dudit mandrin démontable (108, 170, 214, 258, 296),
ladite sonde MPS (110, 172, 216, 254, 294) étant destinée à être reliée à un MPS (102, 162), ledit MPS (102, 162) étant destiné à être relié à un générateur de champ électromagnétique (104, 166), ledit générateur de champ électromagnétique (104, 166) générant un champ électromagnétique, ladite sonde MPS (110, 172, 216, 254, 294) produisant un résultat selon ledit champ électromagnétique, ledit MPS (102, 162) déterminant la position dudit sommet dudit mandrin démontable (108, 170, 214, 258, 296) dans un système de coordonnées correspondant audit MPS (102, 162), selon ledit résultat de ladite sonde MPS (110, 172, 216, 254, 294), ledit MPS (102, 162) produisant une indication correspondant à ladite position, pour permettre une navigation de ladite aiguille chirurgicale (106, 174, 212, 252, 292) vers un organe cible,
**caractérisé en ce que**
ladite sonde MPS (110, 172, 216, 254, 294) est fermement reliée au sommet dudit mandrin (108,170,214,258,296) démontable de telle sorte que ladite sonde MPS (110, 172, 216, 254, 294) est étanche vis-à-vis de ladite paroi intérieure (136, 222, 262, 304) dudit lumen (126, 224, 264, 306), par laquelle l'entrée de substances corporelles indésirables dans l'aiguille chirurgicale (106, 174, 212, 252, 292) est bloquée, pendant que ladite aiguille chirurgicale (106, 174, 212, 252, 292) est entrain d'être avancée vers ledit organe cible.

2. Le dispositif selon la revendication 1, dans lequel l'aiguille chirurgicale (106, 174, 212, 252, 292) est adaptée pour prélever un échantillon d'un fluide corporel à partir dudit organe cible.

3. Le dispositif selon l'une quelconque des revendications 1 ou 2, dans lequel ladite aiguille chirurgicale (106, 174, 212, 252, 292) est adaptée pour injecter une substance thérapeutique dans ledit organe cible.

4. Le dispositif selon l'une quelconque des revendications précédentes, dans lequel ladite aiguille chirurgicale est jetable.

5. Le dispositif selon l'une quelconque des revendications précédentes, dans lequel ladite sonde MPS est située à l'intérieur d'un boitier MPS, ledit boitier MPS étant de la forme d'un cylindre, dans lequel ledit boitier MPS est fermement relié audit sommet du mandrin démontable (108, 170, 214, 258, 296).

6. Le dispositif selon l'une quelconque des revendications 1 à 4, dans lequel ladite sonde MPS est située dans un boitier MPS, ledit boitier MPS étant de la forme d'un tube, et dans lequel ledit boitier MPS est fermement relié à une périphérie externe dudit mandrin démontable (108, 170, 214, 258, 296), à une extrémité distale dudit mandrin démontable (108, 170, 214, 258,296).

7. Le dispositif selon la revendication 6, dans lequel un diamètre interne dudit boitier MPS est substantiellement égal à un diamètre externe dudit mandrin démontable (108, 170, 214, 258, 296).

8. Le dispositif selon la revendication 6, dans lequel un diamètre externe de boitier (302) dudit boitier MPS est substantiellement égal à un diamètre externe de mandrin dudit mandrin démontable (108, 170, 214, 258, 296),
dans lequel ledit mandrin démontable (108, 170, 214, 258, 296) inclut un dégagement (300) au niveau d'une portion distale de celui-ci, et
dans lequel ledit boitier MPS est logé dans ledit dégagement (300).

9. Le dispositif selon l'une quelconque des revendications 1 à 4, dans lequel ladite sonde MPS (110, 172, 216, 254, 294) est située à l'intérieur d'un boitier MPS, ledit boitier MPS étant de la forme d'un adhésif, couvrant ladite sonde MPS (110, 172, 216, 254, 294).

10. Le dispositif selon l'une quelconque des revendications précédentes, dans lequel le mandrin démontable (108, 170, 214, 258, 296) est de la forme d'une tige solide.

11. Le dispositif selon l'une quelconque des revendications 1 à 9, dans lequel le mandrin démontable (108, 170, 214, 258, 296) est de la forme d'un tube.

12. Le dispositif selon l'une quelconque des revendications 1 à 4, dans lequel ladite sonde MPS est de la forme d'une bobine électriquement conductrice autour d'une surface externe dudit mandrin démontable (108, 170, 214, 258, 296).

13. Le dispositif selon l'une quelconque des revendications précédentes, comprenant en outre une barrière jetable placée au-dessus dudit mandrin démontable (108, 170, 214, 258, 296), ladite barrière jetable réduisant la probabilité d'un transfert d'un virus ou d'une bactérie dudit patient à un autre patient.

14. Le dispositif selon l'une quelconque des revendications précédentes, dans lequel ledit mandrin démontable (108, 170, 214, 258, 296) bloque l'entrée de substances corporelles indésirables dudit corps dudit patient, dans ladite aiguille chirurgicale (106, 174, 212, 252, 292), pendant que ladite aiguille chirurgicale (106, 174, 212, 252, 292) est entrain d'être avancée vers ledit organe cible.

15. Système (100, 160) pour la navigation d'une aiguille chirurgicale (106, 174, 212, 252, 292) vers un organe cible dudit corps d'un patient, le système (100, 160) comprenant :
un dispositif à aiguille chirurgicale selon l'une quelconque des revendications précédentes ;
un générateur de champ électromagnétique (104, 166) pour la génération d'un champ électromagnétique ; et un MPS (102 ; 162) relié à ladite sonde MPS (110, 172, 216, 254, 294) et audit générateur de champ électromagnétique (104, 166), ladite sonde MPS (110, 172, 216, 254, 294) produisant un résultat selon ledit champ électromagnétique, ledit MPS (102, 162) déterminant la position dudit sommet dudit mandrin démontable (108, 170, 214, 258, 296) dans un système de coordonnées correspondant audit MPS (102, 162), selon ledit résultat de ladite sonde MPS (110, 172, 216, 254, 294), ledit MPS (102, 162) produisant une indication correspondant à ladite position, pour permettre la navigation de ladite aiguille chirurgicale (106, 174, 212, 252, 292) vers ledit organe cible.

16. Le système (100, 160) selon la revendication 15, comprenant en outre :
un processeur (116, 180) relié à ladite sonde MPS (110, 172, 216, 254, 294) ;
une source d'image (114) relié audit processeur (116, 180), ladite source d'image (114) incluant une image d'organe dudit organe cible ; et
un affichage (112) relié audit processeur (116, 180),
dans lequel ledit processeur (116, 180) détermine ladite position de ladite sonde MPS (110, 172, 216, 254, 294), selon ledit résultat,
dans lequel ledit processeur (116, 180) produit une superposition d'une représentation de ladite position de ladite image d'organe, et
dans lequel ledit affichage (112) affiche ladite superposition.

17. Le système (100, 160) selon la revendication 16, comprenant en outre un marqueur radio-opaque (256) situé au niveau dudit sommet dudit mandrin démontable (108, 170, 214, 258, 296), dans lequel ledit affichage (112) affiche une image de marqueur dudit marqueur radio-opaque (256) contre une image d'organe dudit organe cible.

18. Le système (100, 160) selon l'une quelconque des revendications 15 à 17, dans lequel ladite sonde MPS (110, 172, 216, 254, 294) est relié audit MPS (102, 162), via un conducteur électrique.

19. Le système (100, 160) selon l'une quelconque des revendications 15 à 17, dans lequel ladite indication est sélectionnée à partir de la liste constituée de : visuelle, sonore et tactile.
